# FASCICULE DE BREVET EUROPEEN

(11) **EP 2 111 843 B1**
(45) Date de publication et mention de la délivrance du brevet: **21.06.2017**
(21) Numéro de dépôt: 09158157.9
(22) Date de dépôt: 17.04.2009
(51) Int. Cl.: A61K 8/368, A61Q 19/10, A61Q 1/14

(54) **Composition cosmétique à effet chauffant comprenant un dérivé d'acide salicylique**
Kosmetische Zusammensetzung mit Erhitzungseffekt, die ein Salicylsäurederivat enthält
Cosmetic composition with heating effect comprising a salicylic acid derivative

(30) Priorité: 25.04.2008 FR 0852805
(43) Date de publication de la demande: 28.10.2009
(73) Titulaire: L'Oréal, 75008 Paris (FR)
(72) Inventeur: Compain, Delphine, 75013 Paris (FR)
(74) Mandataire: Duvert, Sandra

(56) Documents cités:
- EP-A- 1 795 175
- WO-A-01/64176
- US-A1- 2004 208 902
- US-A1- 2006 246 027
- US-A1- 2007 148 112
- US-A1- 2007 149 435

## Description

La présente invention a pour objet une composition cosmétique anhydre, comprenant un composé d'acide salicylique, un sel métallique, et un acide carboxylique , et les utilisations de ladite composition dans le domaine cosmétique notamment pour le nettoyage et/ou le démaquillage et/ou gommage de la peau.

Il est connu d'utiliser des compositions cosmétiques anhydres exothermiques, c'est-à-dire ayant un effet chauffant lors de l'application sur la peau. Les produits chauffants peuvent contenir différentes matières premières, comme par exemple des sels métalliques exothermiques qui, en présence d'eau, dégagent de la chaleur.

Ces compositions chauffantes peuvent comprendre des actifs de soin comme par exemple l'acide salicylique ou ses dérivés. Toutefois, la présence simultanée de sels métalliques exothermiques et d'acide salicylique au sein des ces compositions peut s'accompagner de l'apparition de points rouges qui peuvent apparaître entre 24 heures et 1 mois après la fabrication du produit, la réaction étant en outre accélérée par la chaleur. Ces points rouges sont inesthétiques et nuisent à la stabilité du produit, ce qui est rédhibitoire pour l'utilisateur.

Les inventeurs ont découvert que l'introduction d'un acide dans une composition comprenant un sel métallique exothermique et un composé d'acide salicylique permet d'éviter ces inconvénients.

De façon plus précise, l'invention a pour objet une composition comprenant dans un milieu anhydre, au moins un sel métallique exothermique, au moins un composé d'acide salicylique tel que décrit ci-après et au moins un acide

Cette composition est stable et ne présente pas de points rouges, même après 3 mois à température ambiante ou après passage en étuve à 50°C et a un effet chaud satisfaisant.

La composition de l'invention est exothermique, c'est-à-dire qu'elle a un effet chauffant, et donc que l'utilisateur ressent un échauffement lors de l'application de la dite composition sur la peau humide ou humidifiée. C'est une composition dont la température en présence d'eau (l'eau ajoutée lors de l'utilisation ou bien l'eau présente dans la peau) peut s'élever de plusieurs degrés (un à vingt degrés) de manière instantanée.

La composition selon l'invention est constituée d'un milieu anhydre. On entend ici par « anhydre » un milieu quasiment anhydre, c'est-à-dire comprenant généralement moins de 6 % en poids d'eau, de préférence moins de 4 % en poids d'eau, et encore mieux moins de 1 % en poids d'eau par rapport au poids total de la composition. Elle est de préférence totalement anhydre et donc exempte d'eau.

La composition de l'invention étant une composition cosmétique et donc destinée à une application topique, elle comporte un milieu physiologiquement acceptable, c'est-à-dire compatible avec la peau, les muqueuses et/ou les fibres kératiniques.

La composition de l'invention est homogène et stable dans le temps. Elle se présente généralement sous forme d'un gel translucide à opaque, mais elle peut se présenter aussi sous forme de crème, de pâte et même de poudre si tous les constituants utilisés sont poudreux, ladite poudre pouvant être utilisée telle quelle ou dans une éponge ou bien encore imprégnée sur un support tel qu'une lingette, un pad, un film sec, un patch.

### Composé d'acide salicylique

Le composé d'acide salicylique présent dans la composition selon l'invention est choisi parmi l'acide salicylique et les composés de formule (I) suivante : 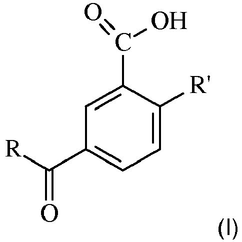 dans laquelle :
- le radical R est un groupement alkyle en C3-C11;
- R' est un groupe hydroxyle ;
- ainsi que leurs sels issus d'une base minérale ou organique.

Parmi les composés de formule (I) particulièrement préférés, on peut citer :
l'acide n-octanoyl-5-salicylique (ou acide capryloyl salicylique) ; l'acide n-décanoyl -5-salicylique ; l'acide n-dodécanoyl-5-salicylique ; et leurs sels correspondants.

Le composé d'acide salicylique est avantageusement choisi parmi l'acide salicylique et l'acide n-octanoyl-5-salicylique, On utilisera plus particulièrement l'acide n-octanoyl-5-salicylique.

Les sels des composés de formule (I) peuvent être obtenus par salification par une base minérale ou organique. A titre d'exemple de base minérale, on peut citer les hydroxydes de métal alcalin ou alcalino-terreux comme l'hydroxyde de sodium, l'hydroxyde de potassium ou l'ammoniaque.

Parmi les bases organiques, on peut citer les amines et les alcanolamines. Les sels quaternaires comme ceux décrits dans le brevet FR 2 607 498 sont particulièrement intéressants.

Les composés de formule (I) utilisables selon l'invention sont décrits dans les brevets US 6,159,479 et US 5,558,871, FR 2,581,542, FR 2 607 498, US4,767,750, EP 378,936, US 5,267,407, US 5,667,789, US 5,580,549, EP-A-570,230.

Le composé d'acide salicylique tel que décrit précédemment peut être présent dans l'émulsion selon l'invention en une teneur allant de 0,01 % à 10 % en poids, par rapport au poids total de la composition, de préférence allant de 0,05 % à 5 % en poids, et préférentiellement allant de 0,05% à 2 % en poids.

### Sels métalliques exothermiques

Les sels métalliques utilisés dans la présente invention sont des sels ayant des propriétés exothermiques, c'est-à-dire dégageant de la chaleur quand ils sont humidifiés. Ils sont choisis en particulier parmi les sels de métal alcalino-terreux, les sels de magnésium et leurs mélanges.

Comme sels de métal alcalino-terreux, on peut citer en particulier les sels de calcium et, plus spécialement, les halogénures de calcium tels que l'iodure, le chlorure et le bromure de calcium. Il s'agit de préférence du chlorure de calcium.

Comme sels de magnésium, on peut citer notamment le sulfate de magnésium.

On peut utiliser aussi un mélange de plusieurs sels.

Selon un mode préféré de réalisation de l'invention, le sel métallique exothermique est choisi parmi le sulfate de magnésium, le chlorure de calcium et leurs mélanges.

La quantité de sel(s) métallique(s) peut aller par exemple de 0,1 à 50 % en poids, de préférence de 5 à 40 % en poids et mieux de 10 à 30 % en poids par rapport au poids total de la composition.

### Acide carboxylique

L'acide carboxylique, composé distinct du composé d'acide salicylique, peut être parmi l'acide lactique, l'acide citrique, l'acide glycolique, l'acide ascorbique, l'acide tartrique et leurs mélanges.

De préférence l'acide carboxylique est choisi parmi l'acide citrique et/ou l'acide glycolique.

L'acide carboxylique peut être présent en une quantité allant de 0,01 à 10 % en poids, de préférence de 0,1 à 5 % en poids et mieux de 0,5 à 3 % en poids par rapport au poids total de la composition.

Selon un mode de réalisation, la composition comprend au moins une silice choisie parmi les silices hydrophile, les silices hydrophobes et leurs mélanges.

La quantité de silice(s) peut aller par exemple de 0,1 à 20 % en poids, de préférence de 0,5 à 10 % en poids et mieux de 1 à 5 % en poids par rapport au poids total de la composition.

On entend par « silice hydrophile » dans la présente demande aussi bien les silices hydrophiles pures que les particules enrobées de silice hydrophile.

Les silices hydrophiles pouvant être utilisées dans la composition de l'invention sont de préférence amorphes, et elles peuvent être d'origine pyrogénée ou d'origine précipitée. Elles se présentent généralement sous forme pulvérulente.

Les silices pyrogénées sont obtenues par pyrolyse en flamme continue à 1000°C du tétrachlorure de silicium (SiCl₄) en présence d'hydrogène et d'oxygène.

Les silices précipitées sont obtenues par réaction d'un acide sur des solutions de silicates alcalins, de préférence du silicate de soude.

Selon un mode préféré de réalisation de l'invention, la silice hydrophile est choisie parmi les silices ayant une surface spécifique de 30 à 500 m²/g, et une dimension moyenne de particules en nombre allant de 3 à 50 nm. Ce sont plus particulièrement les silices hydrophiles décrites dans les tableaux (1) et (2) ci-dessous, et leurs mélanges.

**Tableau (1)**

| Nom commercial | AEROSIL 90 (Société Degussa-Hüls) | AEROSIL 130 (Société Degussa-Hüls) | AEROSIL 150 (Société Degussa-Hüls) | AEROSIL 200 (Société Degussa-Hüls) |
|---|---|---|---|---|
| Mode d'obtention | Pyrogénation | Pyrogénation | Pyrogénation | Pyrogénation |
| Surface BET (m²/g) | 90± 15 | 130± 25 | 150± 15 | 200± 15 |
| Dimension moyenne des particules (nm) | 20 | 16 | 14 | 12 |
| Remarque | | | | taille des agrégats : 10-30 et 200 µ |

**Tableau (2)**

| Nom commercial | AEROSIL 300 (Société Degussa-Hüls) | AEROSIL 380 (Société Degussa-Hüls) | AEROSIL OX 50 (Société Degussa-Hüls) | SILICE FK 320 DS (Société Degussa-Hüls |
|---|---|---|---|---|
| Mode d'obtention | Pyrogénation | Pyrogénation | Pyrogénation | Précipitation |
| Surface BET (m²/g) | 300± 30 | 380± 30 | 50± 25 | 170± 25 |
| Grandeur moyen des particules (nm) | 7 | 7 | 40 | 18 |

La silice hydrophile utilisable dans la composition de l'invention peut consister également en une particule recouverte totalement ou partiellement de silice, notamment d'une particule minérale recouverte totalement ou partiellement de silice, telle que les billes de silice contenant de l'oxyde de titane, commercialisées sous la dénomination TORAYCERAM S-IT® par la société Toray ; les micro-sphères de silice-alumine contenant de l'oxyde de titane (taille : 105 µ), commercialisées sous la dénomination Z-LIGHT-SPHERE W 1012® par la société Zeelan ; les particules de silice synthétique précipitée amorphe/oxyde de titane (taille : 106-500 µ), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 µ), commercialisée sous la dénomination ACS-0050510® par la société SACI-CFPA ; le nano-oxyde de titane anatase traité alumine et silice à 40% dans l'eau (taille : 60 nm, monodisperse), commercialisé sous la dénomination MIRASUN TIW 60® par la société Rhodia Chimie CRA ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie CRA ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane : 30 nm ; épaisseur de silice : 2 nm) commercialisé sous la dénomination MAXLIGHT TS-042® par la société Nichimen Europe PLC.

On utilise de préférence comme silice hydrophile, les silices pyrogénées et en particulier celles commercialisées sous les dénominations AEROSIL (nom INCI : silica) et notamment celle commercialisée sous la dénomination AEROSIL 200 par la société Degussa-Hüls.

La quantité de silice(s) hydrophile(s) peut aller par exemple de 0,1 à 10 % en poids, de préférence de 0,5 à 5 % en poids et mieux de 1 à 3 % en poids par rapport au poids total de la composition.

On entend par « silice hydrophobe » dans la présente demande aussi bien les silices hydrophobes pures que les particules enrobées de silice hydrophobe.

Les silices hydrophobes pouvant être utilisées dans la composition de l'invention sont de préférence amorphes et d'origine pyrogénée. Elles se présentent de préférence sous forme pulvérulente.

Les silices hydrophobes amorphes d'origine pyrogénée sont obtenues à partir de silices hydrophiles. Ces dernières sont obtenues par pyrolyse en flamme continue à 1000°C du tétrachlorure de silicium (SiCl₄) en présence d'hydrogène et d'oxygène. Elles sont ensuite rendues hydrophobes par un traitement avec des silanes halogénés, des alcoxysilanes ou des silazanes. Les silices hydrophobes diffèrent des silices de départ hydrophiles par, entre autres, une densité de groupe silanols plus faible et par une adsorption de vapeur d'eau plus petite.

Selon un mode préféré de réalisation de l'invention, la silice hydrophobe est choisie parmi les silices ayant une surface spécifique de 50 à 500 m²/g, et une dimension moyenne de particules en nombre allant de 3 à 50 nm. Ce sont plus particulièrement les silices hydrophobes décrites dans le tableau (3) ci-dessous, et leurs mélanges.

**Tableau (3)**

| Nom commercial | AEROSIL R202 (Société Degussa-Hüls) | AEROSIL R805 (Société Degussa-Hüls) | AEROSIL R812 (Société Degussa-Hüls) | AEROSIL R972 (Société Degussa-Hüls) | AEROSIL R974 (Société Degussa-Hüls) |
|---|---|---|---|---|---|
| Surface BET (m²/g) | 90 ± 20 | 150 ± 25 | 260 ± 30 | 110 ± 20 | 170 ± 20 |
| Dimension moyenne des particules (nm) | 14 | 12 | 7 | 16 | 12 |

La silice hydrophobe utilisée dans la composition de l'invention peut consister également en une particule recouverte totalement ou partiellement de silice, notamment d'une particule minérale recouverte totalement ou partiellement de silice hydrophobe, telle que les pigments et oxydes métalliques recouverts de silice hydrophobe. Ces particules peuvent aussi avoir des propriétés optiques dans le produit comme sur la peau, par exemple elles peuvent avoir un effet matifiant ou légèrement blanchissant.

On utilise de préférence comme silice hydrophobe, une silice pyrogénée hydrophobe traitée en surface par un dimethylsiloxane, comme celle commercialisée sous la dénomination AEROSIL R974 (nom INCI : Silica Dimethyl Silylate) par la société Degussa-Hüls.

La quantité de silice(s) hydrophobe(s) peut aller par exemple de 0,1 à 10 % en poids, de préférence de 0,5 à 5 % en poids et mieux de 1 à 5 % en poids par rapport au poids total de la composition.

Selon un mode préféré de réalisation de l'invention, la composition de l'invention contient en outre au moins un polyol car les polyols complètent l'action exothermique des sels.

A titre de polyols, on peut citer notamment les polyols ayant au moins 2 groupes hydroxyle et au moins 3 atomes de carbone, tels que la glycérine, la diglycérine, et les glycols tels que le propylène glycol, le dipropylène glycol, le butylène glycol, l'hexylène glycol, le polyéthylène glycol et les polyéthylène glycols de poids moléculaire inférieur à 600 comme le PEG-8 (ou Polyethylene 400), les sucres tels que le sorbitol, et leurs mélanges.

Comme polyols, on utilise de préférence la glycérine, le butylène glycol, le propylène glycol, le dipropylène glycol, le PEG-8 et leurs mélanges.

La quantité de polyol(s) dépend de la quantité de sels présents dans la composition et l'effet chauffant recherché. Elle peut aller par exemple de 0,5 à 80 % en poids, de préférence de 5 à 75 % en poids, mieux de 10 à 70 % en poids et encore mieux de 20 à 65 % en poids er encore mieux de 30 à 60 % en poids par rapport au poids total de la composition.

### Milieu anhydre

La composition selon l'invention comporte de préférence un milieu huileux comprenant au moins une huile. La quantité d'huile(s) peut aller par exemple de 5 à 80 % en poids, de préférence de 10 à 60 % en poids, mieux de 10 à 40 % en poids par rapport au poids total de la composition.

On entend par "huile" un corps gras liquide à la température ambiante (25°C).

Comme huiles utilisables dans la composition de l'invention, on peut citer par exemple :
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de coriandre, de pépins de raisin, de sésame, de noisette, d'abricot (Prunus Armenica Oil), de macadamia, d'arara, de rUniqeman, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin ou 660084 PCL-LIQUID de la société SYMRISE (mélange d'éthyl-2 hexanoate de cétylstéaryle et de myristate d'isopropyle), l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane vendu sous la dénomination "MSX 4518^{®}" par la Société 3M et le nonafluoroéthoxyisobutane ; les dérivés de perfluoromorpholine, tels que la 4-trifluorométhyl perfluoromorpholine vendue sous la dénomination "PF 5052^{®}" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyl-diphényl trisiloxanes, les 2-phényléthyltriméthyl-siloxysilicates, et les polyméthyl-phénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

Quand la composition de l'invention est utilisée comme composition démaquillante, elle contient de préférence au moins une huile démaquillante, à laquelle on peut ajouter une ou plusieurs autres huiles démaquillantes ou non. Les huiles démaquillantes peuvent être choisies notamment parmi les hydrocarbures ramifiés d'origine minérale tels que le polyisobutène hydrogéné, les esters d'acide gras décrits ci-dessus, et leurs mélanges. Comme esters d'acide gras utilisables comme huiles démaquillantes, on peut citer plus particulièrement le palmitate d'éthyl hexyle, le stéarate d'éthyl hexyle, le myristate d'isopropyle, le palmitate d'isopropyle, le palmitate d'isobutyle, le caprate/caprylate de pentaérythritol, l'isononanoate de cétéaryle, l'isononanoate d'isodécyle, l'isononanoate d'isononyle, l'isononanoate d'isotridécyle, le caprate/caprylate d'éthyl-2 hexyle, et leurs mélanges.

En plus des huiles indiquées ci-dessus, la composition de l'invention peut contenir d'autres corps gras, tels que les acides gras comportant de 8 à 30 atomes de carbone, comme l'acide stéarique ; les résines de silicone telles que la trifluorométhyl-C1-4-alkyldimethicone et la trifluoropropyldimethicone ; les gommes de silicone (nom INCI : Dimethiconol) seules ou en mélange avec une huile de silicone, telles que le produit commercialisé par la société Dow Corning sous la dénomination Dow Corning 1501 Fluid, qui est un mélange de Dimethiconol et de Cyclopentasiloxane en proportion 14,7 / 85,3. (dimethiconol) ; les élastomères de silicone tels que ceux commercialisés sous les dénominations KSG par la société Shin-Etsu ; les cires, par exemple les cires minérales, les cires d'origine animale comme la cire d'abeille, les cires d'origine végétale, les huiles hydrogénées concrètes à 25 °C, les esters gras et les glycérides concrets à 25 °C, les cires synthétiques telles que la cire de polyméthylène, les cires de silicone ; et leurs mélanges.

### Additifs

La composition peut, en outre, contenir un ou plusieurs autres additifs, notamment lipophiles, classiquement utilisés dans les compositions cosmétiques, en particulier dans les compositions de nettoyage ou de démaquillage.

On peut citer notamment les composés exothermiques autres que les sels et les polyols, comme par exemple les zéolithes qui sont des silicoaluminates. A titre de zéolites, on peut citer notamment les zéolites activées, et par exemple les zéolites A, les zéolites X comme celles commercialisées par les sociétés Fluka et Union Carbide, les zéolites MAP telles que décrites dans le document EP-A-384070, les zéolites A activées telles que décrites dans le document EP-A-187912 et notamment celles commercialisées sous le nom de ADVERA 401 N ET ADVERA 402N par la société PQ Corporation.

On peut aussi citer comme additifs, les tensioactifs ; les parfums ; les conservateurs ; les antioxydants ; les séquestrants ; les charges ; les colorants, les actifs cosmétiques ou dermatologiques, ou leurs mélanges. Ces adjuvants sont utilisés dans les proportions habituelles des compositions nettoyantes et/ou de soin, et par exemple de 0,01 à 10 % du poids total de la composition. Ces adjuvants doivent être de nature et utilisés en quantité telles qu'ils ne perturbent pas les propriétés recherchées pour la composition de l'invention.

Selon un mode particulier de réalisation de l'invention, la composition selon l'invention contient comme charges, des particules exfoliantes qui vont permettre le gommage de la peau. Comme particules exfoliantes, on peut utiliser des particules exfoliantes ou gommantes d'origines minérale, végétale ou organique. Ainsi, on peut utiliser par exemple les billes ou la poudre de polyéthylène, comme celles commercialisées sous la dénomination Microthene MN 727 ou Microthene MN 710-20 par la société Equistar ou comme la poudre commercialisée sous la dénomination Gotalene 120 Incolore 2 par la société Dupont ; les particules de Nylon comme celles commercialisées par la société Arkema sous la dénomination Orgasol 2002 EXD NAT COS ; les fibres comme les fibres de polyamide, comme celles commercialisées par la société Utexbel sous la dénomination PULPE POLYAMIDE 12185 TAILLE 0,3 MM ; la poudre de polychlorure de vinyle ; la pierre ponce (nom INCI : pumice) comme le ponce 3/B d'Eyraud ; les coques de noyaux de fruits broyées comme les broyats de noyaux d'abricots ou de coques de noix ; la sciure de bois ; les billes de verre ; l'alumine (oxyde d'aluminium) (nom INCI: Alumina) comme le produit commercialisé sous la dénomination Dermagrain 900 par la société Marketech International, ;les cristaux de sucre ; des billes qui fondent lors de l'application sur la peau, telles que par exemple, les sphères à base de mannitol et cellulose commercialisées sous les dénominations Unisphères par la société Induchem, les capsules à base d'agar commercialisées sous les dénominations Primasponge par la société Cognis, et les sphères à base d'esters de jojoba commercialisées sous les dénominations Florasphères par la société Floratech ; et leurs mélanges.

La composition selon l'invention peut aussi contenir d'autres charges telles que, par exemple, le talc, l'amidon modifié ou non, et notamment les amidons estérifiés par l'anhydride octénylsuccinique et plus particulièrement le "Aluminium Starch octenyl succinate" tel que le produit commercialisé par la société National Starch sous le nom de Dry-Flo,.

Ces charges (exfoliantes et autres) peuvent être présentes en une quantité allant par exemple de 0,5 à 20 % en poids, de préférence de 1 à 15 % en poids, mieux de 1 à 10 % et encore mieux de 2 à 5 % en poids par rapport au poids total de la composition.

Comme actifs, on peut citer tout actif de soin ou de nettoyage habituellement utilisé dans le domaine cosmétique, et en particulier des antibactériens comme l'octopirox et le triclosan, des agents kératolytiques, des huiles essentielles, des vitamines comme la vitamine C (acide ascorbique, la vitamine A (rétinol), la vitamine PP (niacinamide), la vitamine B3 (panthénol) et leurs dérivés.

La composition de l'invention est particulièrement adaptée pour le nettoyage et/ou le démaquillage de la peau et/ou des muqueuses et plus particulièrement pour le nettoyage et/ou le démaquillage et/ou le gommage de la peau.

L'invention a encore pour objet l'utilisation cosmétique de la composition telle que définie ci-dessus, pour le nettoyage et/ou le démaquillage et/ou le gommage de la peau.

La composition de l'invention se présente généralement sous forme d'un gel. Lors de l'utilisation, on applique la composition sur la peau (notamment la peau du visage), de préférence en couche épaisse, puis on humidifie les mains et on masse le visage avec les mains mouillées. On peut éventuellement réhumidifier les mains et masser à nouveau le visage. On termine en rinçant le visage. Un autre procédé consiste à humidifier d'abord la peau, puis à appliquer la composition sur la peau humide et à masser le visage, après avoir ou non humidifié les mains.

Ainsi, l'invention a encore pour objet un procédé cosmétique de démaquillage et/ou de nettoyage et/ou de gommage de la peau, consistant à humidifier la peau, à appliquer sur la peau, la composition telle que définie ci-dessus, à masser et à rincer la peau. L'invention a aussi pour objet un procédé cosmétique de démaquillage et/ou de nettoyage de la peau, consistant à appliquer sur la peau, la composition telle que définie ci-dessus, à mouiller les mains, à masser le visage avec les mains mouillées, et à rincer la peau.

Les exemples ci-après sont donnés à titre illustratif et non limitatif en vue de mieux faire ressortir les caractéristiques de l'invention. Les quantités y sont données en % en poids.

### Exemple 1 : Composition chauffante nettoyante

| | | |
|---|---|---|
| - Acide n-octanoyl 5-salicylique (Mexoryl SAB de Chimex) | 0,3 | % |
| - PEG-8 | 53,70 | % |
| - Acide citrique | 1 | % |
| - Glycérine | 5 | % |
| - Silice hydrophile (AEROSIL 200) | 2 | % |
| - Silice hydrophobe (AEROSIL R974) | 1 | % |
| - Cyclopentasiloxane / Dimethiconol (Dow Corning 1501 Fluid) (liant) | 15 | % |
| - Mélange sulfate de magnésium et eau (98/2) | 20 | % |

Mode opératoire : On a mélangé le PEG-8, l'acide citrique, l'acide n--octanoyl 5-salicylique et la glycérine dans un bêcher et on a chauffé le mélange jusqu'à 75°C ( jusqu'à dissolution complète des poudre), on y a ajouté la silice hydrophobe et la silice hydrophile en mélangeant jusqu'à obtention d'un gel lisse, puis le sulfate de magnésium. On a homogénéisé le mélange au Raynerie puis on a jouté le Cyclopentasiloxane / Dimethiconol.

On obtient un gel blanchâtre qui donne un effet chaud bien perceptible et permet d'obtenir un bon nettoyage de la peau.

Après plus de 6 mois à température ambiante, la composition reste stable et sans points rouges.

La composition est divisée en 4 échantillons qui sont placés pendant 2 mois à respectivement 4°C, 25°C, 37°C et 45°C : ces échantillons restent stables.

### Exemple 2 : Composition chauffante nettoyante

| | | |
|---|---|---|
| - Acide n-octanoyl 5-salicylique (Mexoryl SAB de Chimex) | 0,3 | % |
| - PEG-8 | 53,70 | % |
| - Acide glycolique (Glypure 99 de Dupont) | 1 | % |
| - Glycérine | 5 | % |
| - Silice hydrophile (AEROSIL 200) | 2 | % |
| - Silice hydrophobe (AEROSIL R974) | 1 | % |
| - Cyclopentasiloxane / Dimethiconol (Dow Corning 1501 Fluid) (liant) | 15 | % |
| - Mélange sulfate de magnésium et eau (98/2) | 20 | % |
| - Alumine (Dermagrain 900) | 2 | % |

Le mode opératoire est analogue à celui de l'exemple 1.

Le produit obtenu est un gel blanchâtre, qui donne un effet chaud bien perceptible et permet d'obtenir un bon nettoyage de la peau.

Après plus de 6 mois à température ambiante, la composition reste stable et sans points rouges.

La composition est divisée en 4 échantillons qui sont placés pendant 2 mois à respectivement 4°C, 25°C, 37°C et 45°C : ces échantillons restent stables.

### Exemples comparatifs 3 à 6

On a préparé les compositions suivantes :

| | **Exemple 3 comparatif** | **Exemple 4 comparatif** | **Exemple 5 selon l'invention** | **Exemple 6 selon l'invention** |
|---|---|---|---|---|
| **Acide n-octanoyl 5-salicylique (Mexoryl SAB de Chimex)** | - | **0,3** | **0,3** | **0,3** |
| **Acide glycolique (Glypure 99 de Dupont)** | - | - | - | 1 |
| Acide **citrique** | - | - | **1** | - |
| Silice hydrophile (AEROSIL 200) | 1 | 1 | 1 | 1 |
| Silice hydrophobe (AEROSIL R974) | 2 | 2 | 2 | 2 |
| Frustules de diatomées (60-200 microns) | - | 0,2 | - | - |
| Billes de jojoba (250-600 microns) (Florabeads jojoba 28/60 Sunburst Orange de Floratech) | - | 0.25 | - | - |
| Poudre de polyéthylène (Gotalene 120 Incolore 23 de Dupont) | 1,5 | - | | |
| Poudre de polyéthylène colorée (Gotalene 120 Bleu 23 de Dupont) | 2 | - | - | - |
| Cyclopentasiloxane / Dimethiconol (Dow Corning 1501 Fluid) | 15 | 15 | 15 | 15 |
| PEG-8 | qsp 100 | qsp 100 | qsp 100 | qsp 100 |
| PEG-7 caprylic/capric glycerides | 2 | 2 | 2 | 2 |
| Mélange sulfate de magnésium et eau (98/2) | 20 | 20 | 20 | 20 |
| Glycérol | 5 | 5 | 5 | 5 |

| **Aspect** | **Exemple 3 comparatif** | **Exemple 4 comparatif** | **Exemple 5 selon l'invention** | **Exemple 6 selon l'invention** |
|---|---|---|---|---|
| A t = 24h après fabrication | pas de points rouges | apparition de points rouges après 24h à TA* | pas de points rouges | pas de points rouges |
| après plusieurs mois | pas de points rouges | points rouges | pas de points rouges | pas de points rouges |

| | | | | |
|---|---|---|---|---|
| *Température ambiante (20-25°C) | | | | |

La présence d'acide glycolique ou citrique permet bien d'empêcher la formation de points rouges liés à la présence de composés d'acide salicylique dans la composition.

## Revendications

1. Composition cosmétique comprenant dans un milieu anhydre, au moins un sel métallique exothermique, au moins un acide carboxylique choisi parmi l'acide lactique, l'acide citrique, l'acide glycolique, l'acide ascorbique, l'acide tartrique et leurs mélanges et au moins un composé d'acide salicylique choisi parmi l'acide salicylique et les dérivés d'acide salicylique de formule (I) : 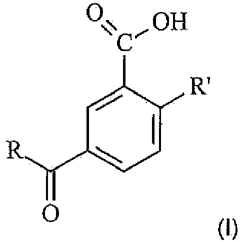 dans laquelle :
- le radical R est un groupement alkyle en C₃-C₁₁;
- R' est un groupe hydroxyle ;
ainsi que leurs sels issus d'une base minérale ou organique.

2. Composition selon la revendication 1, **caractérisée par le fait que** le composé de formule (I) est choisi parmi l'acide n-octanoyl-5-salicylique (ou acide capryloyl salicylique) ; l'acide n-décanoyl -5-salicylique ; l'acide n-dodécanoyl-5-salicylique ; et leurs sels correspondants.

3. Composition selon l'une quelconque des revendications précédentes, **caractérisée par le fait que** le composé d'acide salicylique est l'acide n-octanoyl 5-salicylique.

4. Composition selon l'une quelconque des revendications précédentes, **caractérisée par le fait que** le composé d'acide salicylique est présent en une teneur allant de 0,05 % à 10 % en poids, par rapport au poids total de la composition, de préférence allant de 0,05 % à 5 % en poids, et préférentiellement allant de 1 % à 4 % en poids.

5. Composition selon l'une quelconque des revendications précédentes, **caractérisée en ce que** le sel métallique exothermique est choisi parmi les sels de métal alcalino-terreux, les sels de magnésium et leurs mélanges.

6. Composition selon l'une quelconque des revendications précédentes, **caractérisée en ce que** le sel métallique exothermique est choisi parmi les halogénures de calcium.

7. Composition selon l'une quelconque des revendications précédentes, **caractérisée en ce que** le sel métallique exothermique est choisi parmi le sulfate de magnésium, le chlorure de calcium et leurs mélanges.

8. Composition selon l'une quelconque des revendications précédentes, **caractérisée en ce que** la quantité de sel(s) métallique(s) exothermique(s) va de 0,1 à 50 % en poids par rapport au poids total de la composition.

9. Composition selon l'une quelconque des revendications précédentes, **caractérisée en ce que** l'acide carboxylique est choisi parmi l'acide citrique et/ou l'acide glycolique.

10. Composition selon l'une quelconque des revendications précédentes, **caractérisée en ce que** l'acide carboxylique est présent en une quantité allant de 0,01 à 10% en poids, de préférence de 0,1 à 5 % en poids et mieux de 0,5 à 3 % en poids par rapport au poids total de la composition.

11. Utilisation cosmétique de la composition selon l'une des revendications précédentes pour le nettoyage et/ou le démaquillage et/ou le gommage de la peau.

12. Procédé cosmétique de démaquillage et/ou de nettoyage et/ou de gommage de la peau, consistant à humidifier la peau, à appliquer sur la peau la composition selon l'une quelconque des revendications 1 à 10, à masser et à rincer la peau.

## Patentansprüche

1. Kosmetische Zusammensetzung, umfassend in einem wasserfreien Medium mindestens ein exothermes Metallsalz, mindestens eine Carbonsäure, die aus Milchsäure, Zitronensäure, Glycolsäure, Ascorbinsäure, Weinsäure und Mischungen davon ausgewählt ist, und mindestens eine Salicylsäureverbindung, die aus Salicylsäure und Salicylsäurederivaten der Formel (I): 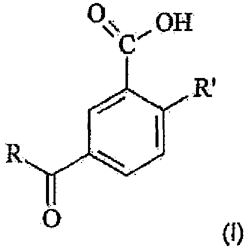 in der:
- der Rest R für eine C₃-C₁₁-Alkylgruppe steht;
- R' für eine Hydroxylgruppe steht;
sowie Salzen davon mit einer anorganischen oder organischen Base ausgewählt ist.

2. Zusammensetzung nach Anspruch 1, **dadurch gekennzeichnet, dass** die Verbindung der Formel (I) aus 5-n-Octanoylsalicylsäure (oder Capryloylsalicylsäure); 5-n-Decanoylsalicylsäure; 5-n-Dodecanoylsalicylsäure und den entsprechenden Salzen davon ausgewählt ist.

3. Zusammensetzung nach einem der vorhergehenden Ansprüche, **dadurch gekennzeichnet, dass** es sich bei der Salicylsäureverbindung um 5-n-Octanoylsalicylsäure handelt.

4. Zusammensetzung nach einem der vorhergehenden Ansprüche, **dadurch gekennzeichnet, dass** die Salicylsäureverbindung in einer Menge im Bereich von 0,05 bis 10 Gew.-%, bezogen auf das Gesamtgewicht der Zusammensetzung, vorzugsweise im Bereich von 0,05 bis 5 Gew.-% und bevorzugt im Bereich von 1 bis 4 Gew.-%, vorliegt.

5. Zusammensetzung nach einem der vorhergehenden Ansprüche, **dadurch gekennzeichnet, dass** das exotherme Metallsalz aus Erdalkalimetallsalzen, Magnesiumsalzen und Mischungen davon ausgewählt ist.

6. Zusammensetzung nach einem der vorhergehenden Ansprüche, **dadurch gekennzeichnet, dass** das exotherme Metallsalz aus Calciumhalogeniden ausgewählt ist.

7. Zusammensetzung nach einem der vorhergehenden Ansprüche, **dadurch gekennzeichnet, dass** das exotherme Metallsalz aus Magnesiumsulfat, Calciumchlorid und Mischungen davon ausgewählt ist.

8. Zusammensetzung nach einem der vorhergehenden Ansprüche, **dadurch gekennzeichnet, dass** die Menge des exothermen Metallsalzes bzw. der exothermen Metallsalze im Bereich von 0,1 bis 50 Gew.-%, bezogen auf das Gesamtgewicht der Zusammensetzung, legt.

9. Zusammensetzung nach einem der vorhergehenden Ansprüche, dadurch gekenntzeichnet, dass die Carbonsäure aus Citronensäure und/oder Glykolsäure ausgewählt ist.

10. Zusammensetzung nach einem der vorhergehenden Anspruche, **dadurch gekennzeichnet, dass** die Carbonsäure in einer Menge im Bereich von 0,01 bis 10 Gew.-%, vorzugsweise von 0,1 bis 5 Gew.-% und noch besser von 0,5 bis 3 Gew.-%, bezogen auf das Gesamtgewicht der Zusammensetzung, vorliegt.

11. Kosmetische Verwendung der Zusammensetzung nach einem der vorhergehenden Ansprüche zum Reinigen und/oder Abschminken und/oder Peeling der Haut.

12. Kosmetisches Verfahren zum Reinigen und/oder zum Abschminken und/oder Peeling der Haut, das darin besteht, dass man die Haut befeuchtet, die Zusammensetzung nach einem der Ansprüche 1 bis 10 auf die Haut aufbringt und die Haut massiert und äbspült.

## Claims

1. Cosmetic composition comprising in an anhydrous medium at least one exothermic metal salt, at least one carboxylic acid chosen from lactic acid, citric acid, glycolic acid, ascorbic acid, tartaric acid and mixtures thereof, and at least one salicylic acid compound chosen from salicylic acid and the salicylic acid derivatives of formula (I): 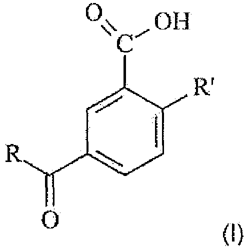 in which:
- the radical is a C₃-C₁₁ alkyl group;
- R' is a hydroxyl group;
and their salts derived from an inorganic or organic base.

2. Composition according to Claim 1, **characterized in that** the compound of formula (I) is chosen from 5-(n-octanoyl)salicylic acid (or capryloylsalicylic acid); 5-(n-decanoyl)salicylic acid; 5-(n-dodecanoyl)salicylic acid and their corresponding salts.

3. Composition according to either one of the preceding claims, **characterized in that** the salicylic acid compound is 5-(n-octanoyl)salicylic acid.

4. Composition according to any one of the preceding claims, **characterized in that** the salicylic acid compound is present in an amount ranging from 0.05% to 10% by weight, relative to the total weight of the composition, preferably ranging from 0.05% to 5% by weight, and more preferably ranging from 1% to 4% by weight.

5. Composition according to any one of the preceding claims, **characterized in that** the exothermic metal salt is chosen from alkaline-earth metal salts, magnesium salts and mixtures thereof.

6. Composition according to any one of the preceding claims, **characterized in that** the exothermic metal salt is chosen from calcium halides.

7. Composition according to any one of the preceding claims, **characterized in that** the exothermic metal salt is chosen from magnesium sulphate, calcium chloride and mixtures thereof.

8. Composition according to any one of the preceding claims, **characterized in that** the quantity of exothermic metal salt(s) ranges from 0.1 to 50% by weight relative to the total weight of the composition.

9. Composition according to any one of the preceding claims, **characterized in that** the carboxylic acid is chosen from citric acid arid/or glycolic acid.

10. Composition according to any one of the preceding claims, **characterized in that** the carboxylic acid is present in a quantity ranging from 0.01 to 10% by weight, preferably from 0.1 to 5% by weight and even better from 0.5 to 3% by weight relative to the total weight of the composition.

11. Cosmetic use of the composition according to one of the preceding claims for cleansing and/or removing makeup from and/or scrubbing the skin.

12. Cosmetic method for removing makeup from and/or cleansing and/or scrubbing the skin, consisting in wetting the skin, in applying to the skin the composition according to any one of Claims 1 to 10, in massaging and in rinsing the skein.
